(19) Europäisches Patentamt
European Patent Office
Office européen des brevets

(11) EP 0 782 987 A1

(12) **EUROPEAN PATENT APPLICATION**
published in accordance with Art. 158(3) EPC

(43) Date of publication:
**09.07.1997 Bulletin 1997/28**

(21) Application number: **96923102.6**

(22) Date of filing: **16.07.1996**

(51) Int. Cl.[6]: **C07D 209/30**, C07D 403/06, A61K 31/40, A61K 31/445, A61K 31/495, A61K 31/535

(86) International application number:
**PCT/JP96/01981**

(87) International publication number:
**WO 97/03963 (06.02.1997 Gazette 1997/07)**

(84) Designated Contracting States:
**AT BE CH DE DK ES FI FR GB GR IE IT LI LU MC NL PT SE**

(30) Priority: **18.07.1995 JP 181952/95**

(71) Applicant: **KYOWA HAKKO KOGYO CO., LTD.**
**Chiyoda-ku, Tokyo 100 (JP)**

(72) Inventors:
- **MACHII, Daisuke**
  **Shizuoka 411 (JP)**
- **TAKAI, Haruki**
  **Kanagawa 246 (JP)**
- **SUZUKI, Koji**
  **Shizuoka 411 (JP)**
- **KOSAKA, Nobuo**
  **Shizuoka 411 (JP)**
- **SATO, Soichiro**
  **Shizuoka 411 (JP)**

(74) Representative: **VOSSIUS & PARTNER**
**Siebertstrasse 4**
**81675 München (DE)**

# (54) INDOLE DERIVATIVES

(57) The present invention relates to indole derivatives represented by formula (I):

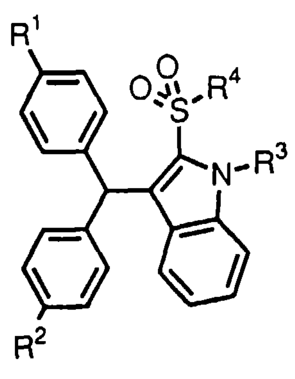

(I)

wherein $R^1$ and $R^2$ independently represent hydrogen, lower alkyl, hydroxy, lower alkoxy, halogen, $-O-(CH_2)_n-OR^5$, or

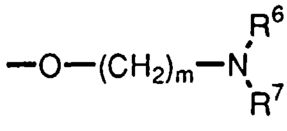

$R^3$ represents hydrogen, lower alkyl, or

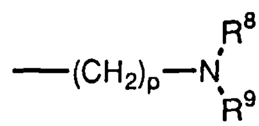

$R^4$ represents hydroxy, lower alkoxy, substituted or unsubstituted aryloxy, or $-NR^{10}R^{11}$, or pharmaceutically acceptable salts thereof.

EP 0 782 987 A1

## Description

### Technical Field

The present invention relates to indole derivatives which are useful as therapeutic agents for osteoporosis.

### Background Art

It is disclosed in Japanese Published Unexamined Patent Application No. 215461/91 that a triphenylmethane derivative having a substituent such as carbamoyl is useful as a therapeutic agent for osteoporosis. Further, it is disclosed in Japanese Published Unexamined Patent Application No. 211651/92 that an indole derivative having a phenyl group is useful as a therapeutic agent for osteoporosis. Further, an indole derivative having a carbamoyl group is disclosed in Japanese Published Unexamined Patent Application No. 76586/95.

### Disclosure of the Invention

The present invention relates to indole derivatives represented by formula (I):

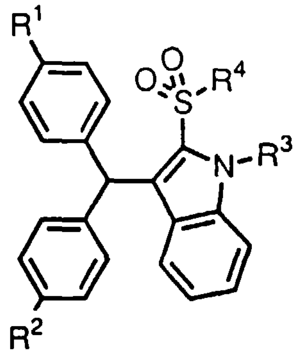

(I)

wherein $R^1$ and $R^2$ independently represent hydrogen, lower alkyl, hydroxy, lower alkoxy, halogen, $-O-(CH_2)_n-OR^5$ (wherein $R^5$ represents hydrogen or lower alkyl, and n represents an integer of 1 to 6), or

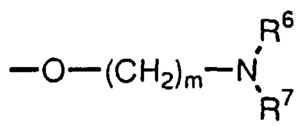

(wherein $R^6$ and $R^7$ independently represent hydrogen or lower alkyl, or $R^6$ and $R^7$ are combined together with the adjacent nitrogen atom to form a substituted or unsubstituted alicyclic heterocyclic group, and m represents an integer of 2 to 6), $R^3$ represents hydrogen, lower alkyl, or

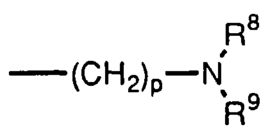

(wherein $R^8$ and $R^9$ independently represent hydrogen or lower alkyl, or $R^8$ and $R^9$ are combined together with the adjacent nitrogen atom to form a substituted or unsubstituted alicyclic heterocyclic group, and p represents an integer of 2 to 6), and $R^4$ represents hydroxy, lower alkoxy, substituted or unsubstituted aryloxy, or $-NR^{10}R^{11}$ {wherein $R^{10}$ and $R^{11}$ independently represent hydrogen, lower alkyl, alicyclic alkyl, substituted or unsubstituted aryl, a substituted or unsubstituted heterocyclic group,

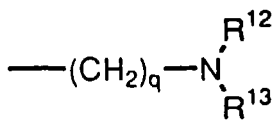

(wherein $R^{12}$ and $R^{13}$ independently represent hydrogen or lower alkyl, or $R^{12}$ and $R^{13}$ are combined together with the adjacent nitrogen atom to form a substituted or unsubstituted alicyclic heterocyclic group, and q represents an integer of 2 to 6), or $-(CH_2)_r-R^{14}$ (wherein $R^{14}$ represents substituted or unsubstituted aryl or a substituted or unsubstituted heterocyclic group, and r represents an integer of 1 to 6), or $R^{10}$ and $R^{11}$ are combined together with the adjacent nitrogen atom to form a substituted or unsubstituted alicyclic heterocyclic group), or pharmaceutically acceptable salts thereof.

The compounds represented by formula (I) are hereinafter referred to as Compound (I). The same applies to the compounds of other formula numbers.

In the definitions of the groups in formula (I), the lower alkyl and the lower alkyl moiety of the lower alkoxy mean a straight or branched alkyl group having 1 to 8 carbon atoms such as methyl, ethyl, propyl, isopropyl, butyl, isobutyl, sec-butyl, tert-butyl, pentyl, neopentyl, hexyl, heptyl, and octyl. The alicyclic alkyl means an alicyclic alkyl group having 3 to 8 carbon atoms such as cyclopropyl, cyclobutyl, cyclopentyl, cyclohexyl, cycloheptyl, and cyclooctyl. The halogen means fluorine, chlorine, bromine, and iodine. The aryl and the aryl moiety of the aryloxy mean phenyl and naphthyl. The alicyclic heterocyclic group means a group such as pyrrolidinyl, imidazolidinyl, pyrazolidinyl, piperidino, piperazinyl, homopiperazinyl, morpholino, and thiomorpholino. The heterocyclic group means a group such as an aromatic heterocyclic group (e.g., pyridyl, pyrazinyl, pyrimidinyl, pyridazinyl, quinolyl, isoquinolyl, phthalazinyl, naphthylidinyl, quinoxalinyl, thienyl, furyl, pyrrolyl, imidazolyl, pyrazolyl, triazolyl, tetrazolyl, thiazolyl, oxazolyl, indolyl, indazolyl, benzimidazolyl, purinyl), pyranyl, piperidyl, and tetrahydrofuranyl, in addition to the above-mentioned alicyclic heterocyclic groups.

The substituted alicyclic heterocyclic group, the substituted heterocyclic group, the substituted aryloxy, and the substituted aryl each has 1 to 3 independently selected substituents. Examples of the substituents are lower alkyl, hydroxy, lower alkoxy, lower alkylthio, aralkyl, carboxy, lower alkoxycarbonyl, lower alkanoyl, aroyl, halogen, nitro, amino, mono- or di(lower alkyl)amino, trifluromethyl, oxo, substituted or unsubstituted phenyl, pyridyl, and pyrimidinyl.

In the definitions of the substituents, the lower alkyl and the lower alkyl moiety of the lower alkoxy, lower alkylthio, lower alkoxycarbonyl, and mono- or di(lower alkyl)amino have the same meaning as defined for the above-mentioned lower alkyl. The aralkyl means an aralkyl group having 7 to 13 carbon atoms such as benzyl, phenethyl, benzhydryl, and naphthylmethyl. The lower alkanoyl means an alkanoyl group having 1 to 7 carbon atoms such as formyl, acetyl, propionyl, butyryl, isobutyryl, valeryl, pivaloyl, hexanoyl, and heptanoyl. The aryl moiety of the aroyl has the same meaning as defined for the above-mentioned aryl. The halogen has the same meaning as defined for the above-mentioned halogen. The substituted phenyl has 1 to 3 independently selected substituents such as lower alkyl, hydroxy, lower alkoxy, lower alkylthio, aralkyl, carboxy, lower alkoxycarbonyl, lower alkanoyl, aroyl, halogen, nitro, amino, mono- or di(lower alkyl)amino, and trifluoromethyl. The lower alkyl, lower alkoxy, lower alkylthio, aralkyl, lower alkoxycarbonyl, lower alkanoyl, aroyl, halogen, and mono- or di(lower alkyl)amino have the same definitions as defined above.

The pharmaceutically acceptable salts of Compound (I) include inorganic acid addition salts such as hydrochloride, sulfate, nitrate, and phosphate, organic acid addition salts such as acetate, maleate, fumarate, tartrate, citrate, lactate, glyoxylate, aspartate, methanesulfonate, ethanesulfonate, and benzenesulfonate, metal salts such as sodium salt, potassium salt, and calcium salt, ammonium salt, tetramethylammonium salt, and amine addition salts such as a salt with morpholine.

The processes for producing Compound (I) are described below.

Compound (I) can be prepared according to the following reaction step:

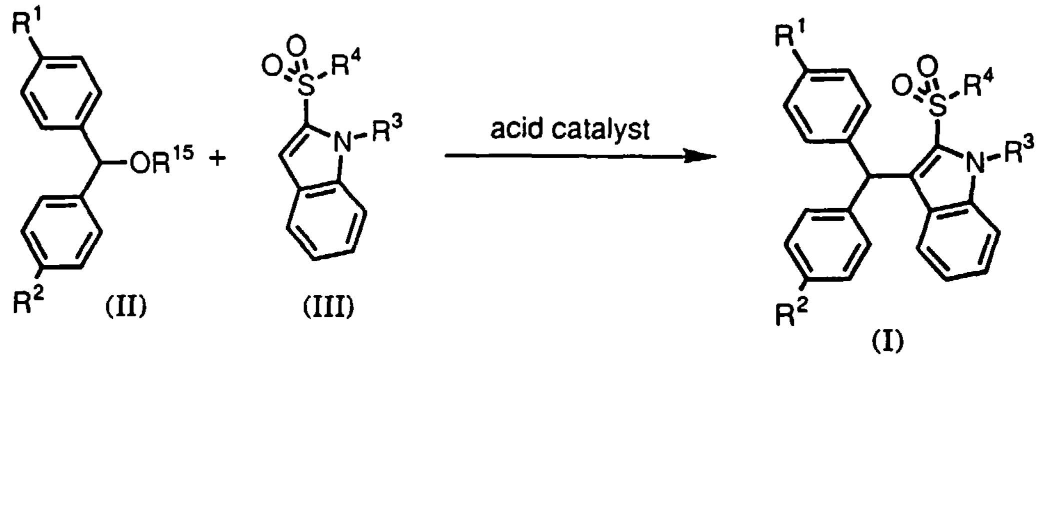


(II) (III) (I)

{In the formulae, $R^{15}$ represents hydrogen, lower alkyl, lower alkanoyl, or

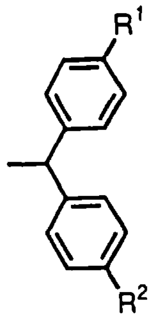

(wherein $R^1$ and $R^2$ have the same meanings as defined above), and $R^1$, $R^2$, $R^3$, and $R^4$ have the same meanings as defined above.}

The lower alkyl and lower alkanoyl in the definition of $R^{15}$ have the same meanings as defined above.

Compound (I) can be obtained by reacting Compound (II) with Compound (III) in the presence of 0.1 equivalence to an excess amount of an acid catalyst such as boron trifluoride-ether complex, methanesulfonic acid, paratoluenesulfonic acid, and trifluoroacetic acid, in a solvent such as methylene chloride, chloroform, ether, and tetrahydrofuran, at a temperature between -20°C and the boiling point of the employed solvent for 0.5 to 48 hours.

Compound (Ib), which is Compound (I) in which $R^1$ and $R^2$ are groups other than hydroxy, and $R^3$ is a group other than hydrogen, can also be prepared from Compound (Ia), which is Compound (I) in which $R^1$ and $R^2$ are groups other than hydroxy, and $R^3$ is hydrogen, according to the following reaction step:

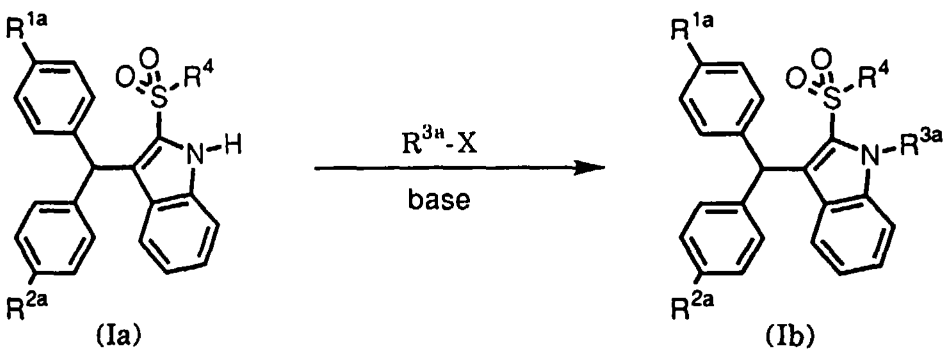


(Ia) (Ib)

(In the formulae, $R^{1a}$ and $R^{2a}$ each represents a group other than hydroxy in the definition of $R^1$ and $R^2$, $R^{3a}$ represents a group other than hydrogen in the definition of $R^3$, X represents chlorine, bromine, or iodine, and $R^4$ has the same meaning as defined above.)

Compound (Ib) can be obtained by reacting Compound (Ia) with a halogenated alkyl or halogenated aminoalkyl in the presence of a base such as sodium hydride and potassium tert-butoxide, in a solvent such as a lower alcohol (e.g., methanol, ethanol), N,N-dimethylformamide, dimethyl sulfoxide, and tetrahydrofuran, at a temperature between 0°C and the boiling point of the employed solvent for 0.5 to 24 hours.

Compound (Id), which is Compound (I) in which both of $R^1$ and $R^2$ are hydroxy, can be prepared from Compound (Ic), which is Compound (I) in which both of $R^1$ and $R^2$ are methoxymethoxy, according to the following reaction step:

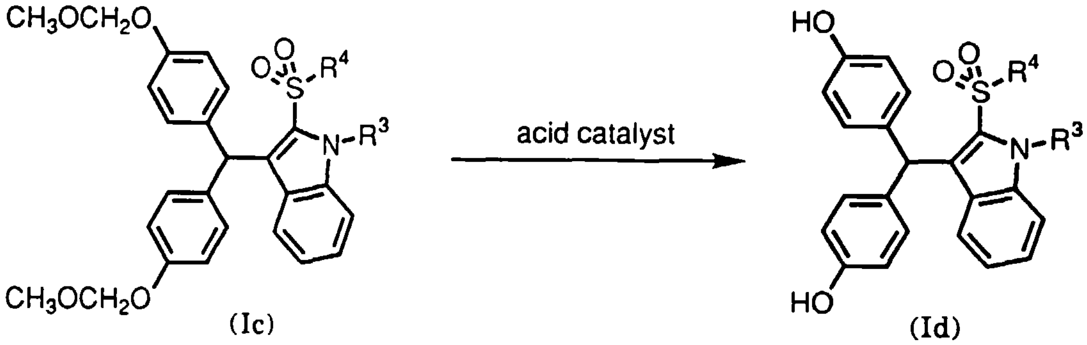


(Ic) (Id)

(In the formulae, $R^3$ and $R^4$ have the same meanings as defined above.)

Compound (Id) can be obtained by treating Compound (Ic) in the presence of an acid catalyst such as hydrochloric acid, sulfuric acid, and paratoluenesulfonic acid, in a solvent such as a lower alcohol (e.g., methanol, ethanol), N,N-dimethylformamide, dimethyl sulfoxide, tetrahydrofuran, and water, or a mixed solvent thereof, at a temperature between room temperature and the boiling point of the employed solvent for 0.5 to 24 hours.

Compound (Ie), which is Compound (I) in which $R^4$ is $-NHR^{10}$ can also be prepared according to the following reaction step:

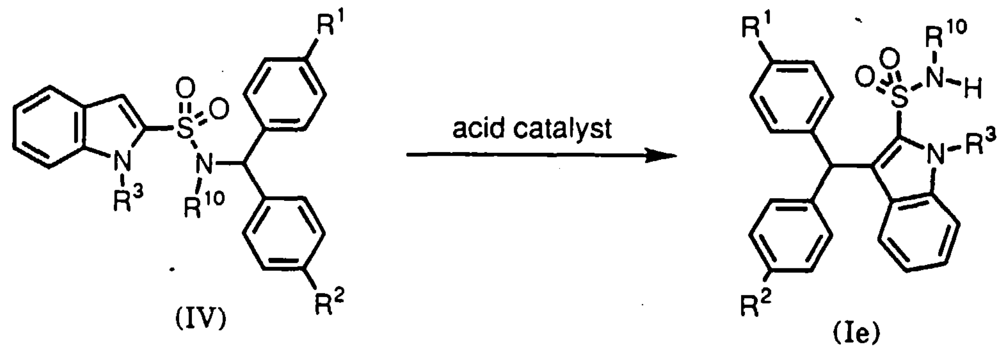


(IV) (Ie)

(In the formulae, $R^1$, $R^2$, $R^3$, and $R^{10}$ have the same meanings as defined above.)

Compound (Ie) can be obtained by treating Compound (IV) in the presence of 0.1 equivalence to an excess amount of an acid catalyst such as boron trifluoride-ether complex, methanesulfonic acid, paratoluenesulfonic acid, and trifluoroacetic acid, in a solvent such as methylene chloride, chloroform, ether, and tetrahydrofuran, at a temperature between -20°C and the boiling point of the employed solvent for 0.5 to 48 hours.

As for the starting compounds, Compound (II) can be obtained according to the method described in Tetrahedron Lett., 28, 5651 (1987) or a similar method thereto, and Compound (III) can be obtained according to the method described in J. Med. Chem., 33, 749 (1990) or a similar method thereto.

The starting Compound (IV) can be prepared from Compound (1) or Compound (2), which are both obtained according to a method similar to that in producing Compound (III), according to the following reaction steps:

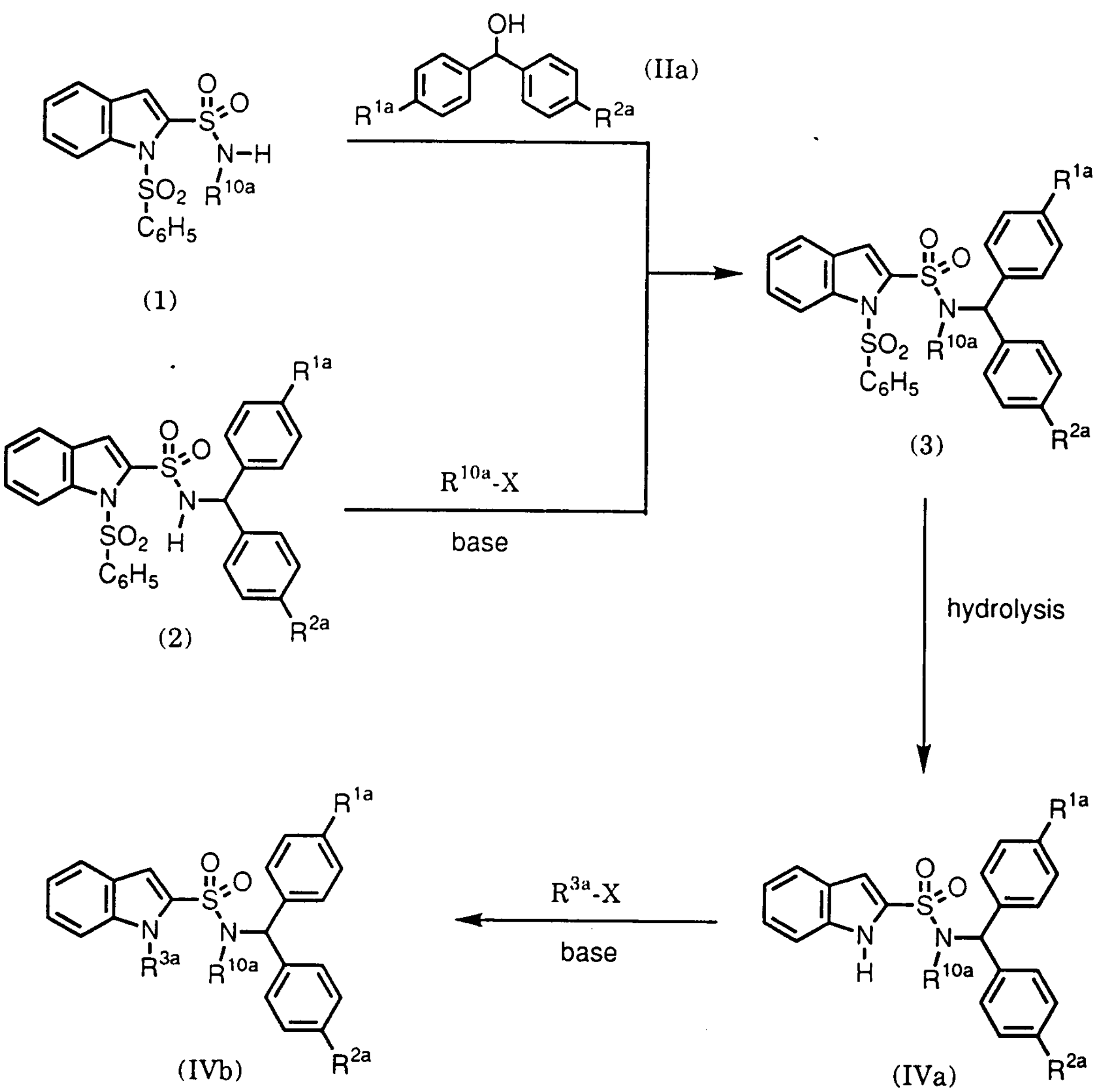


(1) (IIa) (2) (3) (IVa) (IVb)

(In the formulae, $R^{10a}$ represents a group other than hydrogen in the definition of $R^{10}$, and $R^{1a}$, $R^{2a}$, $R^{3a}$, and X have the same meanings as defined above.)

Compound (3) can be obtained by reacting Compound (1) with Compound (IIa), which is Compound (II) in which $R^{1a}$ and $R^{2a}$ are groups other than hydroxy, and $R^{15}$ is hydrogen, in the presence of triphenylphosphine and the like, and diethyl azodicarboxylate and the like, in a solvent such as tetrahydrofuran, at a temperature between 0°C and the boiling point of the employed solvent for 0.5 to 48 hours. Compound (3) can also be obtained from Compound (2) according to a method similar to that in producing Compound (Ib) from Compound (Ia). Compound (IVa) can be obtained from Compound (3) under the normal hydrolytic condition, for example, treating Compound (3) in the presence of a base such as lithium hydroxide, sodium hydroxide, and potassium hydroxide, in a solvent such as a lower alcohol (e.g., methanol, ethanol), tetrahydrofuran, N,N-dimethylformamide, and dimethyl sulfoxide, if necessary, in the presence of water, at a temperature between room temperature and the boiling point of the employed solvent for 0.5 to 24 hours. Then, Compound (IVb) can be obtained from Compound (IVa) according to a method similar to that in producing Compound (Ib) from Compound (Ia).

The intermediates and the desired compounds in the processes described above can be isolated and purified by purification methods conventionally used in organic synthetic chemistry, for example, filtration, extraction, washing, drying, concentration, recrystallization, and various kinds of chromatography. The intermediates may also be subjected to the subsequent reaction without purification.

In the case where a salt of Compound (I) is desired and it is produced in the form of the desired salt, it can be subjected to purification as such. In the case where Compound (I) is produced in the free state and its salt is desired, Compound (I) is dissolved or suspended in a suitable solvent, followed by addition of an appropriate acid or base to form a salt, and then the salt can be isolated.

Compounds (I) and pharmaceutically acceptable salts thereof may be in the form of adducts with water or various solvents, which are also within the scope of the present invention.

Examples of Compounds (I) obtained in the above processes are shown in Table 1.

Table 1-1

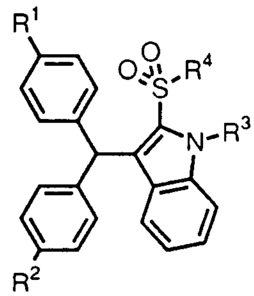

| Compd. No. | $R^1$ | $R^2$ | $R^3$ | $-R^4$ |
|---|---|---|---|---|
| 1 | $OCH_2OCH_3$ | $OCH_2OCH_3$ | H | 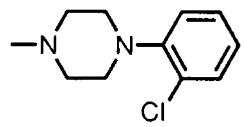 |
| 2 | $OCH_2OCH_3$ | $OCH_2OCH_3$ | 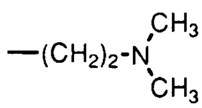 | 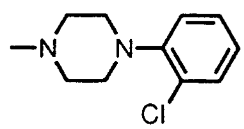 |
| 3 | $OCH_2OCH_3$ | $OCH_2OCH_3$ | 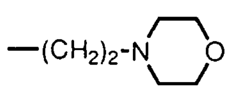 | 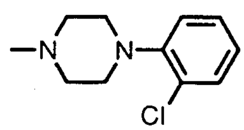 |
| 4 | $OCH_2OCH_3$ | $OCH_2OCH_3$ | H | 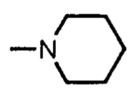 |
| 5 | $OCH_2OCH_3$ | $OCH_2OCH_3$ | H | 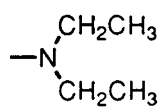 |
| 6 | $OCH_2OCH_3$ | $OCH_2OCH_3$ | H | 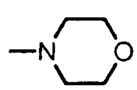 |
| 7 | $OCH_2OCH_3$ | $OCH_2OCH_3$ | H | 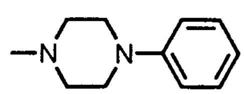 |

Table 1-2

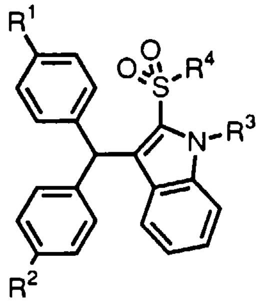

| Compd. No. | $R^1$ | $R^2$ | $R^3$ | $-R^4$ |
|---|---|---|---|---|
| 8 | $OCH_2OCH_3$ | $OCH_2OCH_3$ | H | 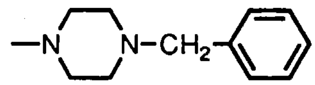 |
| 9 | $OCH_2OCH_3$ | $OCH_2OCH_3$ | $CH_3$<br>$-(CH_2)_2-N$<br>$CH_3$ | 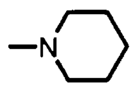 |
| 10 | $OCH_2OCH_3$ | $OCH_2OCH_3$ | $CH_3$<br>$-(CH_2)_2-N$<br>$CH_3$ | $CH_2CH_3$<br>$-N$<br>$CH_2CH_3$ |
| 11 | $OCH_2OCH_3$ | $OCH_2OCH_3$ | $CH_3$<br>$-(CH_2)_2-N$<br>$CH_3$ | 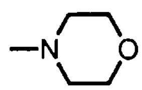 |
| 12 | $OCH_2OCH_3$ | $OCH_2OCH_3$ | $CH_3$<br>$-(CH_2)_2-N$<br>$CH_3$ | 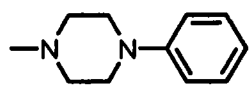 |
| 13 | $OCH_2OCH_3$ | $OCH_2OCH_3$ | $CH_3$<br>$-(CH_2)_2-N$<br>$CH_3$ | 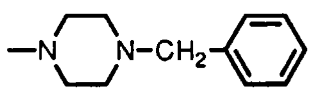 |

Table 1-3

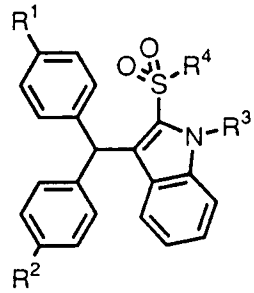

| Compd. No. | $R^1$ | $R^2$ | $R^3$ | $-R^4$ |
|---|---|---|---|---|
| 14 | OH | OH | H | 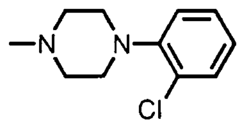 |
| 15 | OH | OH | 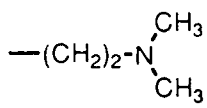 | 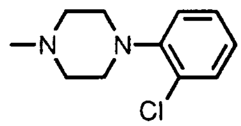 |
| 16 | OH | OH | 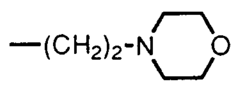 | 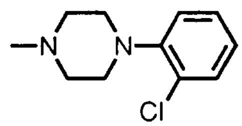 |
| 17 | OH | OH | 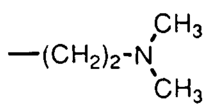 | 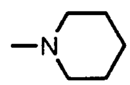 |
| 18 | OH | OH | 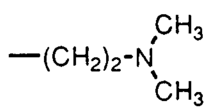 | 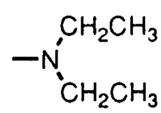 |
| 19 | OH | OH | 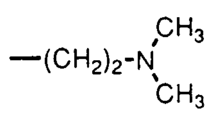 | 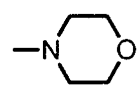 |
| 20 | OH | OH | 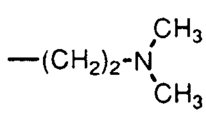 | 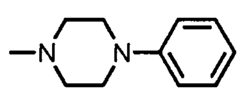 |

Table 1-4

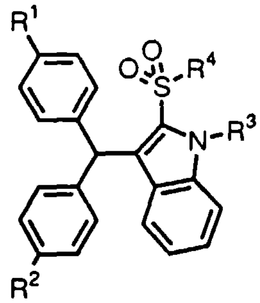

| Compd. No. | $R^1$ | $R^2$ | $R^3$ | $-R^4$ |
|---|---|---|---|---|
| 21 | OH | OH | 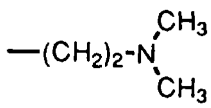 | 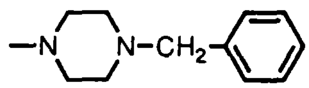 |
| 22 | H | H | H | 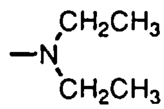 |
| 23 | H | H | H | 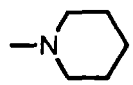 |
| 24 | H | H | 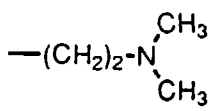 | 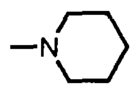 |

Table 1-5

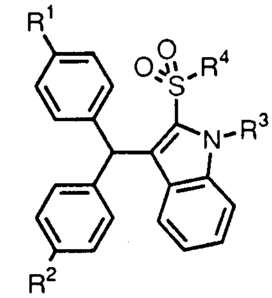

| Compd. No. | $R^1$ | $R^2$ | $R^3$ | $-R^4$ |
|---|---|---|---|---|
| 25 | H | H | H | 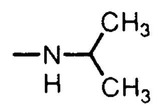 |
| 26 | $CH_3$ | $CH_3$ | H | 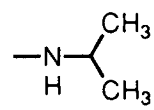 |
| 27 | F | F | H | 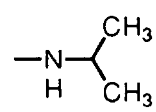 |
| 28 | Cl | Cl | H | 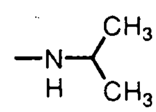 |
| 29 | $OCH_2OCH_3$ | $OCH_2OCH_3$ | H | 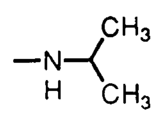 |
| 30 | OH | OH | H | 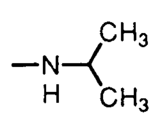 |
| 31 | OH | H | H | 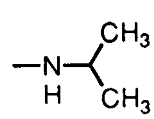 |

Table 1-6

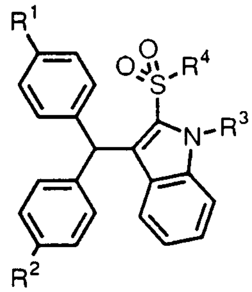

| Compd. No. | $R^1$ | $R^2$ | $R^3$ | $-R^4$ |
|---|---|---|---|---|
| 32 | 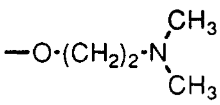 | H | H | 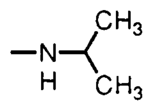 |
| 33 | H | H | 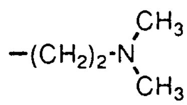 | 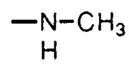 |
| 34 | H | H | 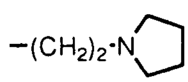 | 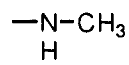 |
| 35 | H | H | 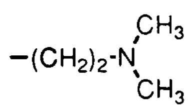 | 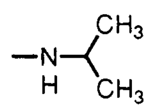 |
| 36 | H | H | 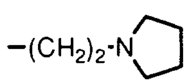 | 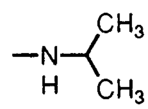 |
| 37 | H | H | 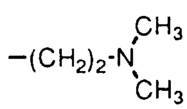 | 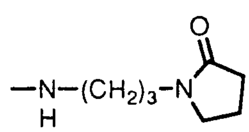 |
| 38 | H | H | 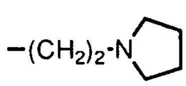 | 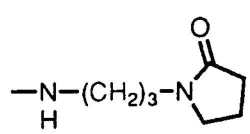 |

The inhibitory effect of the compounds of the present invention on bone absorption is shown below by test examples.

Test Example 1: Inhibitory effect on bone absorption

The calvaria was excised from a newborn dd mouse (5 to 6 days old) under sterile conditions. The calvaria was washed with a modified Dulbecco phosphate buffer physiological saline solution containing neither calcium nor magnesium (a product of Gibco Oriental), and divided into two parts along the center suture. A half of the calvaria was cultivated in a modified Dulbecco Eagle medium (1.5 ml) (a product of Gibco Oriental) containing 2.5% fetal calf serum and 15% equine serum which had been inactivated by heating at 56°C for 20 minutes. The test compound was dissolved in dimethyl sulfoxide and 10 µl of the resulting solution was added to the culture medium to give the final concentrations of $3 \times 10^{-6}$M, $1 \times 10^{-5}$M, and $3 \times 10^{-5}$M. PTH (parathyroid hormone) was dissolved in a 0.15 M saline solution (pH 3) and 3 µl of the resulting solution was added to the culture medium to give the final concentration of $1 \times 10^{-8}$M. The culturing was carried out under the condition of 95% air and 5% carbon dioxide and at a temperature of 37°C for 96 hours. At 48 hours after the start of the culturing, the culture medium was renewed and the test compound and PTH treated as defined above were added thereto. For examining the effect of the test compound on calcium liberation (bone absorption) from the PTH enhanced bone, a control group, a group using PTH ($1 \times 10^{-8}$M), and a group using both the test compound ($3 \times 10^{-6}$M, $1 \times 10^{-5}$M, $3 \times 10^{-5}$M) and PTH were prepared. The amount of the bone absorption was determined by measuring the amount of calcium accumulated in the culture as collected after 96 hours of culturing. The total calcium concentration in the culture was measured with Calcium C Test Wako. The inhibition rate was calculated using the following equation and the result was shown in terms of 50% inhibitory concentration ($IC_{50}$). The results are shown in Table 2.

Inhibition of bone absorption from PTH enhanced bone Inhibition rate (%) = $[(C_P - C_D)/(C_P - C_0)] \times 100$

$C_0$ : The total calcium concentration in the culture containing neither the test compound nor PTH.
$C_P$ : The total calcium concentration in the culture treated with only PTH.
$C_D$ : The total calcium concentration in the culture treated with both the test compound and PTH.

Table 2

| Compound No. Inhibitory | effect on bone absorption ($IC_{50}$;µM) |
|---|---|
| 14 | 14.1 |
| 17 | 13.7 |
| 18 | 13.3 |
| 24 | 13.7 |

The inhibitory effect of the compounds of the present application on bone resorption was also determined by their inhibitory effect on the excretion of urinary hydroxyproline which is elevated by ovariectomy of the animals tested, according to the report of Kalu et al. [Bone and Mineral., 14, 175 (1991)].

Test Example 2: Inhibitory effect on the increase of urinary hydroxyproline excretion by ovariectomy

12-weeks-old female SD strain rats (Japan Clea Co., Ltd.) were acclimated for 1 week during which they were given tap water and a standard diet (F2; Funabashi Farmas) ad libitum. The experiment was carried out using the rats weighing 270-310 g. After the animals were subjected to sham operation or bilateral ovariectomy, they were allowed free access to water passed through ion exchange resin instead of tap water, and were housed in individual cages. The test compound (10 mg/kg) was suspended in 0.3% Tween, and the obtained suspension was orally administered to the ovariectomized rats once a day for two weeks starting from the day following ovariectomy (0.5 ml/100 g body weight). 0.3% Tween was similarly administered to sham-operated rats and ovariectomized control rats. After the final administration, the rats were placed in individual metabolic cages and fasted during 24 hours, and urine was collected. The volume of the collected urine was measured and the urine was centrifuged at 3,000 rpm for 15 min at 4°C. The concentration of hydroxyproline in the supernatant was measured by the method of Ikeda et al. [Ann. Rep. Tokyo Metr. Res. Lab. P. H., 36, 277-282 (1985)] and the concentration of creatinine in the supernatant was measured with creatinine test WAKO

(Wako Pure Chem.) The amount of urinary hydroxyproline excretion was expressed by the molar ratio of the amount of hydroxyproline to the amount of creatinine. The rate of inhibition on increase of the amount of urinary hydroxyproline excretion in ovariectomized rats treated with the test compound compared to that of the control group was calculated using the following equation.

$$\text{Inhibition rate (\%)} = [(P1 - P2)/(P1 - P3)] \times 100$$

P1 : The amount of urinary hydroxyproline excretion in ovariectomized rats treated with 0.3% Tween (μmol/mmol).
P2 : The amount of urinary hydroxyproline excretion in ovariectomized rats treated with the test compound (μmol/mmol).
P3 : The amount of urinary hydroxyproline in sham-operated rats treated with 0.3% Tween (μmol/mmol).

Table 5

| Compd. No. | Inhibition rate of increase of the amount of urinary hydroxyproline excretion |
|---|---|
| 16 | 32 |
| 18 | 22 |
| 24 | 100 |

Compounds (I) and pharmaceutically acceptable salts thereof can be formulated into generally employed dose forms such as tablets, capsules, and syrups, and administered orally or parenterally through intramuscular injection, intravenous injection, drip infusion, or rectal administration using suppositories. For preparing these dose forms for oral or parenteral administration, generally known techniques are applied. For example, the preparations may contain various excipients, lubricants, binders, disintegrating agents, isotonizing agents, emulsifiers, and the like.

Examples of the carriers which can be used are water, injectable distilled water, physiological saline, glucose, fructose, sucrose, mannitol, lactose, starch, cellulose, methyl cellulose, carboxymethyl cellulose, hydroxypropyl cellulose, alginic acid, talc, sodium citrate, calcium carbonate, calcium hydrogenphosphate, magnesium stearate, urea, silicone resins, sorbitan fatty acid esters, and glycerin fatty acid esters.

The effective dose and administration schedule of Compound (I) or a pharmaceutically acceptable salt thereof varies depending upon the mode of administration, the age, body weight and conditions of a patient, etc. However, generally, Compound (I) or a pharmaceutically acceptable salt thereof is administered in a dose of 0.1 to 10 mg/kg/day in 1 to 4 parts.

Certain embodiments of the present invention are illustrated in the following Examples and Reference Examples.

## Best Mode for Carrying out the Invention

### Example 1

3-{Bis[4-(methoxymethoxy)phenyl]methyl}-2-[4-(2-chlorophenyl)piperazinylsulfonyl]indole (Compound 1)

Boron trifluoride-ether complex (0.04 ml, 0.32 mmol) was added to a solution of 2-[4-(2-chlorophenyl)piperazinylsulfonyl]indole (1.2 g, 3.19 mmol) in 15 ml of methylene chloride, and a solution of 4,4'-bis(methoxymethoxy)benzhydrol (991 mg, 3.26 mmol) in 10 ml of methylene chloride was added thereto, followed by stirring at room temperature for 20 hours. Then, a solution of 4,4'-bis(methoxymethoxy)benzhydrol (490 mg, 1.60 mmol) in 10 ml of methylene chloride was further added to the reaction mixture to complete the reaction. A saturated aqueous solution of sodium bicarbonate was added to the reaction solution for neutralization followed by extraction with chloroform. The resulting organic layer was washed with a saturated aqueous solution of sodium bicarbonate and a saturated aqueous solution of sodium chloride, and dried over magnesium sulfate, and the solvent was distilled off under reduced pressure to give a crude product. The obtained crude product was crystallized from diisopropyl ether/hexane to give 1.9 g (yield: 90%) of the title compound.

$^1$H-NMR($CDCl_3$) δ(ppm): 2.8-2.9 (4H, m), 3.05-3.15 (4H, m), 3.44 (6H, s), 5.12 (4H, s), 6.35 (1H, s), 6.85-7.0 (3H, m), 6.93 (4H, d, J=8.9Hz), 7.15-7.25 (2H, m), 7.17 (4H, d, J=8.9Hz), 7.25-7.35 (2H, m), 7.40 (1H, d, J=8.3Hz), 8.71 (1H, br s).

Example 2

3-{Bis[4-(methoxymethoxy)phenyl]methyl}-2-[4-(2-chlorophenyl)piperazinylsulfonyl]-1-(2-dimethylaminoethyl) indole (Compound 2)

To a solution of Compound 1 (1.2 g, 1.81 mmol) obtained in Example 1 in 25 ml of N,N-dimethylformamide was portionwise added sodium hydride (60% in oil, 160 mg, 3.81 mmol) with stirring at 0°C, and 2-dimethylaminoethylchloride hydrochloride (260 mg, 1.81 mmol) was added thereto, followed by heating to 80°C and then stirring for 7 hours. A saturated aqueous solution of ammonium chloride was added to the reaction solution for neutralization, and water was added thereto followed by extraction with ethyl acetate. The resulting organic layer was washed with a saturated aqueous solution of sodium chloride and dried over magnesium sulfate, and the solvent was distilled off under reduced pressure to give a crude product. The obtained crude product was purified with silica gel column chromatography (ethyl acetate/hexane = 2/1) to give 1.1 g (yield: 85%) of the title compound.

$^1$H-NMR($CDCl_3$) δ(ppm): 2.45 (6H, s), 2.75-2.9 (6H, m), 3.1-3.2 (4H, m), 3.43 (6H, s), 4.6-4.7 (2H, m), 5.12 (4H, s), 6.63 (1H, s), 6.85-7.05 (7H, m), 7.05-7.25 (6H, m), 7.3-7.4 (2H, m), 7.47 (1H, d, J=8.4Hz).

Example 3

3-{Bis[4-(methoxymethoxy)phenyl]methyl}-2-[4-(2-chlorophenyl)piperazinylsulfonyl]-1-(2-morpholinoethyl)indole (Compound 3)

Substantially the same procedure as in Example 2 was repeated using Compound 1 (1.1 g, 1.66 mmol) obtained in Example 1 and 2-morpholinoethylchloride hydrochloride (310 mg, 1.66 mmol) to give 1.3 g (quantitative) of the title compound.

$^1$H-NMR($CDCl_3$) δ(ppm): 2.5-2.6 (4H, m), 2.7-2.8 (2H, m), 2.8-2.9 (4H, m), 3.05-3.15 (4H, m), 3.43 (6H, s), 3.65-3.75 (4H, m), 4.55-4.65 (2H, m), 5.12 (4H, s), 6.62 (1H, s), 6.8-7.0 (3H, m), 6.91 (4H, d, J=8.6Hz), 7.1-7.25 (2H, m), 7.13 (4H, d, J=8.6Hz), 7.3-7.4 (2H, m), 7.43 (1H, d, J=8.3Hz).

Example 4

3-{Bis[4-(methoxymethoxy)phenyl]methyl}-2-(piperidinosulfonyl)indole (Compound 4)

To a solution of 2-(piperidinosulfonyl)indole (0.9 g, 3.40 mmol) and bis[4,4'-bis(methoxymethoxy)benzhydryl] ether (1.1 g, 1.87 mmol) in 20 ml of methylene chloride was added boron trifluoride-ether complex (0.04 ml, 0.32 mmol), followed by stirring at room temperature for 2 hours. A saturated aqueous solution of sodium bicarbonate was added to the reaction solution for neutralization followed by extraction with chloroform. The resulting organic layer was washed with a saturated aqueous solution of sodium bicarbonate and a saturated aqueous solution of sodium chloride, and dried over magnesium sulfate, and the solvent was distilled off under reduced pressure to give 2.4 g of a crude product. The obtained crude product was purified with silica gel column chromatography (ethyl acetate/hexane = 1/3) to give 1.5 g (yield: 79%) of the title compound.

$^1$H-NMR($CDCl_3$) δ(ppm): 1.2-1.35 (2H, m), 1.35-1.5 (4H, m), 2.85-2.95 (4H, m), 3.45 (6H, s), 5.13 (4H, s), 6.30 (1H, s), 6.91 (4H, d, J=8.4Hz), 6.9-7.0 (1H, m), 7.05-7.15 (1H, m), 7.14 (4H, d, J=8.4Hz), 7.2-7.3 (1H, m), 7.39 (1H, d, J=7.9Hz), 8.77 (1H, s).

Example 5

N,N-diethyl-3-(bis[4-(methoxymethoxy)phenyl]methyl)indole-2-sulfonamide (Compound 5)

Substantially the same procedure as in Example 4 was repeated using N,N-diethylindole-2-sulfonamide (1.0 g, 3.96 mmol) and bis[4,4'-bis(methoxymethoxy)benzhydryl] ether (1.29 g, 2.18 mmol) to give 1.56 g (yield: 73%) of the title compound.

$^1$H-NMR($CDCl_3$) δ(ppm): 0.94 (6H, t, J=7.2Hz), 3.06 (4H, q, J=7.2Hz), 3.46 (6H, s), 5.13 (4H, s), 6.30 (1H, s), 6.85-6.95 (1H, m), 6.91 (4H, d, J=8.7Hz), 7.05-7.15 (1H, m), 7.12 (4H, d, J=8.7Hz), 7.2-7.3 (1H, m), 7.38 (1H, d, J=8.4Hz), 8.83 (1H, s).

Example 6

3-{Bis[4-(methoxymethoxy)phenyl]methyl}-2-(morpholinosulfonyl)indole (Compound 6)

Substantially the same procedure as in Example 4 was repeated using 2-(morpholinosulfonyl)indole (0.75 g, 2.82 mmol) and bis[4,4'-bis(methoxymethoxy)benzhydryl] ether (0.92 g, 1.55 mmol) to give 1.42 g (yield: 91%) of the title compound.

$^1$H-NMR($CDCl_3$) δ(ppm): 2.85-2.95 (4H, m), 3.45 (6H, s), 3.45-3.55 (4H, m), 5.13 (4H, s), 6.31 (1H, s), 6.9-7.0 (1H, m), 6.92 (4H, d, J=8.4Hz), 7.1-7.2 (1H, m), 7.13 (4H, d, J=8.4Hz), 7.2-7.3 (1H, m), 7.39 (1H, d, J=8.3Hz), 8.75-8.85 (1H, br).

Example 7

3-{Bis[4-(methoxymethoxy)phenyl]methyl}-2-(4-phenylpiperazinylsulfonyl)indole (Compound 7)

Substantially the same procedure as in Example 4 was repeated using 2-(4-phenylpiperazinylsulfonyl)indole (1.0 g, 2.93 mmol) and bis[4,4'-bis(methoxymethoxy)benzhydryl] ether (0.95 g, 1.61 mmol) to give 1.63 g (yield: 89%) of the title compound.

$^1$H-NMR($CDCl_3$) δ(ppm): 3.15-3.25 (8H, m), 3.46 (6H, s), 5.13 (4H, s), 5.34 (1H, s), 6.75 (2H, d, J=8.6Hz), 6.9-7.0 (10H, m), 7.06 (1H, d, J=2.0Hz), 7.21 (1H, t, J=6.9Hz), 7.36 (1H, t, J=6.9Hz), 7.45 (1H, d, J=8.3Hz), 7.70 (1H, d, J=7.3Hz), 8.88 (1H, s).

Example 8

3-{Bis[4-(methoxymethoxy)phenyl]methyl}-2-(4-benzylpiperazinylsulfonyl)indole (Compound 8)

Substantially the same procedure as in Example 4 was repeated using 2-(4-benzylpiperazinylsulfonyl)indole (1.0 g, 2.81 mmol) and bis[4,4'-bis(methoxymethoxy)benzhydryl] ether (0.91 g, 1.55 mmol) to give 1.16 g (yield: 64%) of the title compound.

$^1$H-NMR($CDCl_3$) δ(ppm): 2.2-2.3 (4H, m), 2.9-3.0 (4H, m), 3.36 (2H, s), 3.43 (6H, s), 5.11 (4H, s), 6.30 (1H, s), 6.9-7.0 (1H, m), 6.91 (4H, d, J=8.6Hz), 7.1-7.2 (4H, m), 7.14 (4H, d, J=8.6Hz), 7.2-7.3 (3H, m), 7.37 (1H, d, J=8.2Hz), 8.67 (1H, s).

Example 9

3-{Bis[4-(methoxymethoxy)phenyl]methyl}-1-(2-dimethylaminoethyl)-2-(piperidinosulfonyl)indole (Compound 9)

Substantially the same procedure as in Example 2 was repeated using Compound 4 (1.32 g, 2.40 mmol) obtained in Example 4 and 2-dimethylaminoethylchloride hydrochloride (345 mg, 2.40 mmol) to give 1.1 g (yield: 74%) of the title compound.

$^1$H-NMR($CDCl_3$) δ(ppm): 1.41 (6H, br s), 2.37 (6H, s), 2.65-2.75 (2H, m), 2.9-3.0 (4H, m), 3.44 (6H, s), 4.5-4.6 (2H, m), 5.12 (4H, s), 6.64 (1H, s), 6.91 (4H, d, J=8.2Hz), 6.85-6.95 (1H, m), 7.05-7.15 (1H, m), 7.12 (4H, d, J=8.2Hz), 7.25-7.35 (1H, m), 7.41 (1H, d, J=8.3Hz).

In the following Examples 10 to 13, substantially the same procedure as in Example 9 was repeated using corresponding Compounds 5 to 8 in place of Compound 4 to give the desired compounds.

Example 10

N,N-Diethyl-3-{bis[4-(methoxymethoxy)phenyl]methyl}-1-(2-dimethylaminoethyl)indole-2-sulfonamide (Compound 10)

$^1$H-NMR($CDCl_3$) δ(ppm): 0.91 (6H, t, J=7.3Hz), 2.40 (6H, s), 2.7-2.8 (2H, m), 3.07 (4H, q, J=7.3Hz), 3.46 (6H, s), 4.45-4.55 (2H, m), 5.13 (4H, s), 6.66 (1H, s), 6.85-6.95 (1H, m), 6.90 (4H, d, J=8.5Hz), 7.0-7.1 (1H, m), 7.11 (4H, d, J=8.5Hz), 7.25-7.35 (1H, m), 7.41 (1H, d, J=8.6Hz).

Example 11

3-{Bis[4-(methoxymethoxy)phenyl]methyl}-1-(2-dimethylaminoethyl)-2-(morpholinosulfonyl)indole (Compound 11)

$^1$H-NMR($CDCl_3$) δ(ppm): 2.39 (6H, s), 2.65-2.75 (2H, m), 2.85-2.95 (4H, m), 3.4-3.5 (4H, m), 3.45 (6H, s), 4.5-4.6 (2H, m), 5.13 (4H, s), 6.59 (1H, s), 6.9-7.0 (1H, m), 6.91 (4H, d, J=8.6Hz), 7.05-7.15 (1H, m), 7.10 (4H, d, J=8.6Hz), 7.3-7.4 (1H, m), 7.42 (1H, d, J=8.6Hz).

Example 12

3-{Bis[4-(methoxymethoxy)phenyl]methyl)-1-(2-dimethylaminoethyl)-2-(4-phenylpiperazinylsulfonyl)indole (Compound 12)

$^1$H-NMR($CDCl_3$) δ(ppm): 2.37 (6H, s), 2.65-2.75 (2H, m), 3.15-3.25 (4H, m), 3.3-3.4 (4H, m), 3.46 (6H, s), 4.5-4.6 (2H, m), 5.13 (4H, s), 5.35 (1H, s), 6.78 (2H, d, J=8.9Hz), 6.85-7.0 (10H, m), 7.15-7.25 (2H, m), 7.35-7.45 (2H, m), 7.69 (1H, d, J=8.2Hz).

Example 13

2-(4-Benzylpiperazinylsulfonyl)-3-{bis[4-(methoxymethoxy)phenyl]methyl}-1-(2-dimethylaminoethyl)indole (Compound 13)

$^1$H-NMR($CDCl_3$) δ(ppm): 2.2-2.3 (4H, m), 2.36 (6H, s), 2.65-2.75 (2H, m), 2.9-3.0 (4H, m), 3.39 (2H, s), 3.42 (6H, s), 4.5-4.6 (2H, m), 5.10 (4H, s), 6.60 (1H, s), 6.85-6.95 (1H, m), 6.89 (4H, d, J=8.7Hz), 7.05-7.15 (1H, m), 7.10 (4H, d, J=8.7Hz), 7.2-7.35 (6H, m), 7.40 (1H, d, J=8.3Hz).

Example 14

3-[Bis(4-hydroxyphenyl)methyl]-2-[4-(2-chlorophenyl)piperazinylsulfonyl]indole (Compound 14)

Compound 1 (1.0 g, 1.51 mmol) obtained in Example 1 was dissolved in a mixed solvent of tetrahydrofuran (10 ml) and ethanol (30 ml), and 20 ml of 2 N hydrochloric acid was added thereto, followed by heating under reflux for 2 hours. The solvent was distilled off under reduced pressure, and a saturated aqueous solution of sodium bicarbonate was added thereto for neutralization. The mixture was stirred and the precipitated crystals were collected to give a crude product. The obtained crude product was washed with ethanol and a little amount of hexane to give 0.77 g (yield: 89%) of the title compound.

$^1$H-NMR(DMSO-$d_6$) δ(ppm): 2.75-2.9 (4H, m), 2.9-3.05 (4H, m), 6.16 (1H, s), 6.66 (4H, d, J=8.4Hz), 6.85-7.05 (8H, m), 7.15-7.25 (2H, m), 7.25-7.35 (1H, m), 7.46 (1H, d, J=8.4Hz), 9.21 (2H, s), 11.98 (1H, s).

In the following Examples 15 to 21, substantially the same procedure as in Example 14 was repeated using corresponding Compounds 2, 3, and 9 to 13 in place of Compound 1 to give the desired compounds.

Example 15

3-[Bis(4-hydroxyphenyl)methyl]-2-[4-(2-chlorophenyl)piperazinylsulfonyl]-1-(2-dimethylaminoethyl)indole (Compound 15)

$^1$H-NMR(DMSO-$d_6$) δ(ppm): 2.28 (6H, s), 2.55-2.65 (2H, m), 2.8-2.9 (4H, m), 3.05-3.15 (4H, m), 4.5-4.6 (2H, m), 6.39 (1H, s), 6.65 (4H, d, J=8.6Hz), 6.91 (4H, d, J=8.6Hz), 6.8-6.9 (1H, m), 6.95-7.05 (3H, m), 7.2-7.4 (3H, m), 7.52 (1H, d, J=8.6Hz), 9.15 (2H, s).

Example 16

3-[Bis(4-hydroxyphenyl)methyl]-2-[4-(2-chlorophenyl)piperazinylsulfonyl]-1-(2-morpholinoethyl)indole (Compound 16)

$^1$H-NMR(DMSO-$d_6$) δ(ppm): 2.4-2.55 (4H, m), 2.55-2.65 (2H, m), 2.8-2.9 (4H, m), 3.05-3.15 (4H, m), 3.5-3.65 (4H, m), 4.5-4.65 (2H, m), 6.38 (1H, s), 6.65 (4H, d, J=8.4Hz), 6.90 (4H, d, J=8.4Hz), 6.9-7.1 (4H, m), 7.2-7.4 (3H, m), 7.58 (1H, d, J=8.9Hz), 9.17 (2H, s).

Example 17

3-[Bis(4-hydroxyphenyl)methyl]-1-(2-dimethylaminoethyl)-2-(piperidinosulfonyl)indole (Compound 17)

$^1$H-NMR(DMSO-$d_6$) δ(ppm): 1.42 (6H, br s), 2.29 (6H, s), 2.55-2.65 (2H, m), 2.96 (4H, br s), 4.45-4.55 (2H, m), 6.39 (1H, s), 6.63 (4H, d, J=8.6Hz), 6.85-6.95 (1H, m), 6.88 (4H, d, J=8.6Hz), 6.99 (1H, d, J=8.0Hz), 7.25-7.3 (1H, m),

7.48 (1H, t, J=8.3Hz), 9.10 (2H, s).

Example 18

N,N-Diethyl-3-[bis(4-hydroxyphenyl)methyl]-1-(2-dimethylaminoethyl)indole-2-sulfonamide (Compound 18)

$^1$H-NMR(DMSO-$d_6$) δ(ppm): 0.89 (6H, t, J=6.9Hz), 2.28 (6H, s), 2.5-2.65 (2H, m), 3.08 (4H, q, J=6.9Hz), 4.4-4.5 (2H, m), 6.44 (1H, s), 6.64 (4H, d, J=8.2Hz), 6.8-7.0 (2H, m), 6.87 (4H, d, J=8.2Hz), 7.2-7.3 (1H, m), 7.47 (1H,d, J=8.4Hz), 9.13 (2H, s).

Example 19

3-[Bis(4-hydroxyphenyl)methyl]-1-(2-dimethylaminoethyl)-2-(morpholinosulfonyl)indole (Compound 19)

$^1$H-NMR(DMSO-$d_6$) δ(ppm): 2.27 (6H, s), 2.5-2.65 (2H, m), 2.85-2.95 (4H, m), 3.4-3.5 (4H, m), 4.45-4.55 (2H, m), 6.35 (1H, s), 6.65 (4H, d, J=8.3Hz), 6.88 (4H, d, J=8.3Hz), 6.85-6.95 (1H, m), 7.00 (1H, d, J=8.3Hz), 7.25-7.35 (1H, m), 7.45-7.55 (1H, m), 9.05-9.2 (2H, br).

Example 20

3-[Bis(4-hydroxyphenyl)methyl]-1-(2-dimethylaminoethyl)-2-(4-phenylpiperazinylsulfonyl)indole (Compound 20)

$^1$H-NMR(DMSO-$d_6$) δ(ppm): 2.22 (6H, s), 2.56 (2H, t, J=7.2Hz), 3.1-3.25 (8H, m), 4.50 (2H, t, J=7.2Hz), 5.19 (1H, s), 6.63 (4H, d, J=8.6Hz), 6.7-6.95 (8H, m), 7.1-7.2 (2H, m), 7.3-7.4 (1H, m), 7.60 (1H, d, J=8.6Hz), 7.70 (1H, d, J=7.9Hz), 9.13 (2H, s).

Example 21

2-(4-Benzylpiperazinylsulfonyl)-3-[bis(4-hydroxyphenyl)methyl]-1-(2-dimethylaminoethyl)indole (Compound 21)

$^1$H-NMR($CDCl_3$) δ(ppm): 2.2-2.3 (4H, m), 2.36 (6H, s), 2.6-2.7 (2H, m), 2.95-3.05 (4H, m), 3.40 (2H, s), 4.45-4.55 (2H, m), 6.49 (1H, s), 6.70 (4H, d, J=8.3Hz), 6.89 (1H, t, J=8.1Hz), 6.98 (4H, d, J=8.3Hz), 7.07 (1H, d, J=8.2Hz), 7.15-7.35 (6H, m), 7.38 (1H, d, J=8.9Hz).

Example 22

N,N-Diethyl-3-(diphenylmethyl)indole-2-sulfonamide (Compound 22)

To a solution of benzhydryl acetate (0.77 g, 3.39 mmol) and N,N-diethylindole-2-sulfonamide (0.9 g, 3.39 mmol) in 20 ml of methylene chloride was added methanesulfonic acid (0.7 ml, 10.7 mmol), followed by stirring at room temperature for one hour. A saturated aqueous solution of sodium bicarbonate was added to the reaction solution for neutralization followed by extraction with chloroform. The resulting organic layer was washed with a saturated aqueous solution of sodium bicarbonate and a saturated aqueous solution of sodium chloride, and dried over magnesium sulfate, and the solvent was distilled off under reduced pressure to give a crude product. The obtained crude product was crystallized from diisopropyl ether to give 0.99 g (yield: 67%) of the title compound.

$^1$H-NMR($CDCl_3$) δ(ppm): 0.90 (6H, t, J=7.2Hz), 3.03 (4H, q, J=7.2Hz), 6.42 (1H, s), 6.85-6.95 (1H, s), 7.05-7.3 (12H, m), 7.39 (1H, d, J=8.3Hz), 8.86 (1H, br s).

Example 23

3-(Diphenylmethyl)-2-(piperidinosulfonyl)indole (Compound 23)

Substantially the same procedure as in Example 22 was repeated using benzhydryl acetate (0.78 g, 3.41 mmol) and 2-(piperidinosulfonyl)indole (0.95 g, 3.59 mmol) to give 1.2 g (yield: 75%) of the title compound.

$^1$H-NMR($CDCl_3$) δ(ppm): 1.2-1.3 (2H, m), 1.35-1.45 (4H, m), 2.8-2.9 (4H, m), 6.43 (1H, s), 6.94 (1H, td, J=8.2, 1.0Hz), 7.14 (1H, d, J=7.6Hz), 7.15-7.3 (11H, m), 7.39 (1H, d, J=8.2Hz), 8.75-8.85 (1H, br).

Example 24

1-(2-Dimethylaminoethyl)-3-(diphenylmethyl)-2-(piperidinosulfonyl)indole (Compound 24)

Substantially the same procedure as in Example 2 was repeated using Compound 23 (1.0 g, 2.25 mmol) obtained in Example 23 and 2-dimethylaminoethylchloride hydrochloride (360 mg, 2.47 mmol) to give 0.85 g (yield: 75%) of the title compound.

$^1$H-NMR($CDCl_3$) δ(ppm): 1.3-1.45 (6H, br), 2.40 (6H, s), 2.7-2.8 (2H, m), 2.85-2.95 (4H, m), 4.5-4.6 (2H, m), 6.74 (1H, s), 6.91 (1H, td, J=6.9, 1.0Hz), 7.01 (1H, d, J=8.2Hz), 7.15-7.35 (11H, m), 7.43 (1H, d, J=8.6Hz).

Example 25

N-Isopropyl-3-(diphenylmethyl)indole-2-sulfonamide (Compound 25)

To a solution of benzhydrol (0.58 g, 3.15 mmol) and N-isopropylindole-2-sulfonamide (0.7 g, 3.12 mmol) in 15 ml of methylene chloride was added a solution of methanesulfonic acid (0.2 ml, 3.12 mmol) in 4 ml of methylene chloride, followed by stirring at room temperature for 2.5 hours. A saturated aqueous solution of sodium bicarbonate was added to the reaction solution for neutralization and the solvent was distilled off under reduced pressure, followed by extraction with ethyl acetate. The resulting organic layer was washed with a saturated aqueous solution of sodium chloride and dried over magnesium sulfate, and the solvent was distilled off under reduced pressure to give 1.4 g of a crude product. The obtained crude product was washed with ethanol to give 1.07 g (yield: 85%) of the title compound.

$^1$H-NMR($CDCl_3$) δ(ppm): 0.62 (6H, t, J=6.3Hz), 3.1-3.25 (1H, m), 3.80 (1H, d, J=7.3Hz), 6.42 (1H, s), 6.9-7.2 (2H, m), 7.1-7.35 (11H, m), 7.40 (1H, d, J=8.3Hz), 8.82 (1H, s).

Example 26

N-Isopropyl-3-[bis(4-methylphenyl)methyl]indole-2-sulfonamide (Compound 26)

Substantially the same procedure as in Example 25 was repeated using 4,4'-dimethylbenzhydrol (0.96 g, 4.50 mmol) and N-isopropylindole-2-sulfonamide (1.00 g, 4.46 mmol) to give 1.44 g (yield: 75%) of the title compound.

$^1$H-NMR($CDCl_3$) δ(ppm): 0.54 (6H, d, J=6.3Hz), 2.22 (6H, s), 3.0-3.15 (1H, m), 3.69 (1H, d, J=6.9Hz), 6.22 (1H, s), 6.8-6.9 (2H, m), 6.99 (4H, d, J=8.3Hz), 7.02 (4H, d, J=8.3Hz), 7.1-7.2 (1H, m), 7.29 (1H, d, J=8.3Hz), 8.73 (1H, s).

Example 27

N-Isopropyl-3-[bis(4-fluorophenyl)methyl]indole-2-sulfonamide (Compound 27)

Substantially the same procedure as in Example 25 was repeated using 4,4'-difluorobenzhydrol (0.99 g, 4.50 mmol) and N-isopropylindole-2-sulfonamide (1.00 g, 4.46 mmol) to give 1.56 g (yield: 80%) of the title compound.

$^1$H-NMR($CDCl_3$) δ(ppm): 0.71 (6H, d, J=6.3Hz), 3.15-3.3 (1H, m), 3.96 (1H, d, J=7.3Hz), 6.38 (1H, s), 6.85-7.05 (6H, m), 7.15-7.25 (4H, m), 7.25-7.35 (1H, m), 7.42 (1H, d, J=8.3Hz), 8.87 (1H, s).

Example 28

N-Isopropyl-3-[bis(4-chlorophenyl)methyl]indole-2-sulfonamide (Compound 28)

Substantially the same procedure as in Example 25 was repeated using 4,4'-dichlorobenzhydrol (1.14 g, 4.50 mmol) and N-isopropylindole-2-sulfonamide (1.00 g, 4.46 mmol) to give 1.76 g (yield: 83%) of the title compound.

$^1$H-NMR($CDCl_3$) δ(ppm): 0.73 (6H, d, J=6.3Hz), 3.15-3.3 (1H, m), 3.97 (1H, d, J=7.3Hz), 6.36 (1H, s), 6.9-7.05 (2H, m), 7.15 (4H, d, J=8.6Hz), 7.2-7.35 (5H, m), 7.42 (1H, d, J=8.6Hz), 8.87 (1H, s).

Example 29

N-Isopropyl-3-[bis[4-(methoxymethoxy)phenyl]methyl)indole-2-sulfonamide (Compound 29)

Substantially the same procedure as in Example 4 was repeated using bis[4,4'-bis(methoxymethoxy)-benzhydryl] ether (1.45 g, 2.45 mmol) and N-isopropylindole-2-sulfonamide (1.00 g, 4.46 mmol) to give 1.93 g (yield: 82%) of the title compound.

$^1$H-NMR($CDCl_3$) δ(ppm): 0.67 (6H, d, J=6.6Hz), 3.1-3.25 (1H, m), 3.46 (6H, s), 3.84 (1H, d, J=7.3Hz), 5.15 (4H,

s), 6.30 (1H, s), 6.9-7.0 (6H, m), 7.15 (4H, d, J=8.9Hz), 7.2-7.3 (1H, m), 7.39 (1H, d, J=8.3Hz), 8.81 (1H, s).

Example 30

N-Isopropyl-3-[bis(4-hydroxyphenyl)methyl]indole-2-sulfonamide (Compound 30)

Substantially the same procedure as in Example 14 was repeated using Compound 29 (1.75 g, 3.34 mmol) obtained in Example 29 to give 1.22 g (yield: 84%) of the title compound.

$^1$H-NMR(DMSO-$d_6$) δ(ppm): 0.85 (6H, d, J=6.6Hz), 3.15-3.3 (1H, m), 6.17 (1H, s), 6.63 (4H, d, J=8.3Hz), 6.8-6.9 (1H, m), 6.96 (4H, d, J=8.3Hz), 7.04 (1H, d, J=8.3Hz), 7.1-7.2 (1H, m), 7.35 (1H, d, J=7.6Hz), 7.43 (1H, d, J=8.3Hz), 9.02 (2H, s), 11.51 (1H, s).

Example 31

N-Isopropyl-3-(4-hydroxybenzhydryl)indole-2-sulfonamide (Compound 31)

N-Isopropyl-3-(4-benzyloxybenzhydryl)indole-2-sulfonamide (1.80 g, 3.52 mmol) obtained in Reference Example 1 was dissolved in a mined solvent of ethanol (30 ml) and tetrahydrofuran (10 ml), and 10% palladium on carbon (0.36 g, 50% aqueous) was added thereto, followed by stirring under a hydrogen atmosphere for 4.5 hours. The catalyst was filtered off and the solvent was distilled off under reduced pressure to give 1.63 g of a crude compound. The obtained crude product was crystallized from diethyl ether to give 1.21 g (yield :82%) of the title compound.

$^1$H-NMR(DMSO-$d_6$) δ(ppm): 0.83 (6H, d, J=6.6Hz), 3.15-3.35 (1H, m), 6.26 (1H, s), 6.65 (2H, d, J=7.6Hz), 6.8-7.05 (4H, m), 7.1-7.3 (6H, m), 7.44 (1H, d, J=8.3Hz).

Example 32

N-Isopropyl-3-[4-(2-dimethylaminoethoxy)benzhydryl]indole-2-sulfonamide hydrochloride (Compound 32 hydrochloride)

Substantially the same procedure as in Example 25 was repeated using 4-(2-dimethylaminoethoxy)benzhydrol (1.22 g, 4.50 mmol) and N-isopropylindole-2-sulfonamide (1.00 g, 4.46 mmol) to give 1.90 g (yield: 87%) of a free base of the title compound. This compound (1.5 g) was dissolved in 10 ml of ethanol and the pH of the solution was adjusted to 1 by addition of a solution of hydrogen chloride in ethyl acetate. The solvent was distilled off under reduced pressure followed by crystallization from ethanol to give 1.33 g (yield: 83%) of the title compound.

$^1$H-NMR($CDCl_3$) δ(ppm): 0.78 (3H, d, J=6.6Hz), 0.84 (3H, d, J=6.6Hz), 2.86 (6H, s), 3.15-3.3 (1H, m), 3.3-3.5 (2H, m), 4.1-4.3 (2H, m), 5.33 (1H, d, J=6.9Hz), 6.44 (1H, s), 6.53 (2H, d, J=8.6Hz), 6.85-7.0 (2H, m), 7.07 (2H, d, J=8.6Hz), 7.15-7.35 (6H, m), 7.46 (1H, d, J=8.3Hz), 10.21 (1H, s).

Example 33

N-Methyl-1-(2-dimethylaminoethyl)-3-(diphenylmethyl)indole-2-sulfonamide hydrochloride (Compound 33 hydrochloride)

To a solution of N-diphenylmethyl-N-methyl-1-(2-dimethylaminoethyl)indole-2-sulfonamide (1.97 g, 4.40 mmol) obtained in Reference Example 8 in 20 ml of chloroform was added methanesulfonic acid (0.57 ml, 8.80 mmol), followed by heating under reflux for 8 hours. A saturated aqueous solution of sodium bicarbonate was added to the reaction solution for neutralization followed by extraction with chloroform. The resulting organic layer was washed with a saturated aqueous solution of sodium chloride and dried over magnesium sulfate, and the solvent was distilled off under reduced pressure to give 2.04 g of a crude product. The obtained crude product was purified with silica gel column chromatography (chloroform/methanol = 100/1) followed by crystallization from diethyl ether to give 1.09 g (yield: 55%) of a free base of the title compound. This compound (0.80 g) was dissolved in 10 ml of ethanol and the pH of the solution was adjusted to 1 by addition of a solution of hydrogen chloride in ethyl acetate. Water was added to the mixture followed by crystallization to give 0.52 g (yield: 60%) of the title compound.

$^1$H-NMR($CDCl_3$) δ(ppm): 2.12 (3H, s), 2.73 (6H, s), 3.1-3.25 (2H, m), 4.95-5.1 (2H, m), 6.73 (1H, s), 6.85-6.95 (2H, m), 7.15-7.35 (12H, m), 7.47 (1H, d, J=8.6Hz).

Example 34

N-Methyl-3-(diphenylmethyl)-1-(2-pyrrolidinylethyl)indole-2-sulfonamide hydrochloride (Compound 34 hydrochloride)

Substantially the same procedure as in Example 33 was repeated using N-diphenylmethyl-N-methyl-1-(2-pyrrolidinylethyl)indole-2-sulfonamide (2.12 g, 4.48 mmol) obtained in Reference Example 9 to give 1.07 g (yield: 50%) of a free base of the title compound. Then, substantially the same procedure as in Example 33 was repeated using this compound (0.90 g) to give 0.95 g (yield: 98%) of the title compound.

$^1$H-NMR($CDCl_3$) δ(ppm): 2.0-2.2 (4H, m), 2.16 (3H, d, J=5.3Hz), 2.9-3.1 (2H, m), 3.4-3.5 (2H, m), 3.85-3.95 (2H, m), 5.1-5.2 (2H, m), 6.0-6.1 (1H, m), 6.71 (1H, s), 6.85-6.9 (2H, m), 7.1-7.3 (11H, m), 7.60 (1H, d, J=8.3Hz), 12.26 (1H, br).

Example 35

N-Isopropyl-1-(2-dimethylaminoethyl)-3-(diphenylmethyl)indole-2-sulfonamide hydrochloride (Compound 35 hydrochloride)

Substantially the same procedure as in Example 33 was repeated using N-diphenylmethyl-N-isopropyl-1-(2-dimethylaminoethyl)indole-2-sulfonamide (1.90 g, 3.99 mmol) obtained in Reference Example 10 to give 1.18 g (yield: 62%) of a free base of the title compound. Then, substantially the same procedure as in Example 33 was repeated using this compound (1.00 g) to give 0.84 g (yield: 78%) of the title compound.

$^1$H-NMR($CDCl_3$) δ(ppm): 0.75 (6H, d, J=6.6Hz), 2.75-3.0 (1H, m), 2.96 (6H, s), 3.35-3.45 (2H, m), 5.1-5.2 (2H, m), 6.08 (1H, d, J=8.3Hz), 6.76 (1H, s), 6.85-6.95 (2H, m), 7.1-7.35 (11H, m), 7.64 (1H, d, J=8.3Hz), 12.49 (1H, br).

Example 36

N-Isopropyl-3-(diphenylmethyl)-1-(2-pyrrolidinylethyl)indole-2-sulfonamide hydrochloride (Compound 36 hydrochloride)

Substantially the same procedure as in Example 33 was repeated using N-diphenylmethyl-N-isopropyl-1-(2-pyrrolidinylethyl)indole-2-sulfonamide (2.00 g, 3.99 mmol) obtained in Reference Example 11 to give 1.44 g (yield: 72%) of a free base of the title compound. Then, substantially the same procedure as in Example 33 was repeated using this compound (1.20 g) to give 1.12 g (yield: 87%) of the title compound.

$^1$H-NMR($CDCl_3$) δ(ppm): 0.72 (6H, d, J=6.3Hz), 2.05-2.2 (4H, m), 2.8-3.05 (3H, m), 3.4-3.5 (2H, m), 3.85-4.0 (2H, m), 5.1-5.2 (2H, m), 5.60 (1H, d, J=8.3Hz), 6.74 (1H, s), 6.85-6.95 (2H, m), 7.15-7.35 (11H, m), 7.67 (1H, d, J=8.6Hz).

Example 37

N-[3-(2-Oxopyrrolidinyl)propyl]-1-(2-dimethylaminoethyl)-3-(diphenylmethyl)indole-2-sulfonamide hydrochloride (Compound 37 hydrochloride)

Substantially the same procedure as in Example 33 was repeated using N-diphenylmethyl-N-[3-(2-oxopyrrolidinyl)propyl]-1-(2-dimethylaminoethyl)indole-2-sulfonamide (1.23 g, 2.20 mmol) obtained in Reference Example 12 to give 0.96 g (yield: 78%) of a free base of the title compound. Then, substantially the same procedure as in Example 33 was repeated using this compound (0.96 g) to give 0.70 g (yield: 69%) of the title compound.

$^1$H-NMR($CDCl_3$) δ(ppm): 1.1-1.3 (2H, m), 1.9-2.05 (2H, m), 2.25-2.4 (4H, m), 2.95 (3H, s), 2.97 (3H, s), 3.14 (2H, t, J=6.3Hz), 3.23 (2H, t, J=6.9Hz), 3.4-3.5 (2H, m), 5.05-5.15 (2H, m), 6.76 (1H, s), 6.85-7.0 (2H, m), 7.1-7.45 (12H, m), 7.85 (1H, d, J=8.3Hz), 12.74 (1H, br).

Example 38

N-[3-(2-Oxopyrrolidinyl)propyl]-3-(diphenylmethyl)-1-(2-pyrrolidinylethyl)indole-2-sulfonamide hydrochloride (Compound 38 hydrochloride)

Substantially the same procedure as in Example 33 was repeated using N-diphenylmethyl-N-[3-(2-oxopyrrolidinyl)propyl]-1-(2-pyrrolidinylethyl)indole-2-sulfonamide (1.62 g, 2.77 mmol) obtained in Reference Example 13 to give 0.50 g (yield: 31%) of a free base of the title compound. Then, substantially the same procedure as in Example 33 was repeated using this compound (0.50 g) to give 0.46 g (yield: 86%) of the title compound.

$^1$H-NMR($CDCl_3$) δ(ppm): 1.2-1.35 (2H, m), 1.9-2.0 (2H, m), 2.05-2.2 (2H, m), 2.2-2.55 (6H, m), 3.0-3.2 (2H, m), 3.2-3.45 (4H, m), 3.55-3.7 (2H, m), 3.95-4.1 (2H, m), 5.15-5.3 (2H, m), 6.90 (1H, s), 7.0-7.1 (2H, m), 7.2-7.55 (12H, m), 8.01 (1H, d, J=7.6Hz), 12.80 (1H, br).

Reference Example 1

N-Isopropyl-3-(4-benzyloxybenzhydryl)indole-2-sulfonamide

Substantially the same procedure as in Example 25 was repeated using 4-benzyloxybenzhydrol (1.31 g, 4.50 mmol) and N-isopropylindole-2-sulfonamide (1.00 g, 4.46 mmol) to give 1.92 g (yield: 84%) of the title compound.

$^1$H-NMR($CDCl_3$) δ(ppm): 0.61 (3H, d, J=6.3Hz), 0.63 (3H, d, J=6.3Hz), 3.1-3.25 (1H, m), 3.75-3.85 (1H, m), 5.04 (2H, s), 6.36 (1H, s), 6.85-7.0 (4H, m), 7.14 (2H, d, J=8.6Hz), 7.2-7.45 (12H, m), 8.78 (1H, br).

Reference Example 2

N-Diphenylmethyl-N-methyl-1-benzenesulfonylindole-2-sulfonamide

To a solution of N-diphenylmethyl-1-benzenesulfonylindole-2-sulfonamide (12.40 g, 24.67 mmol) in 120 ml of N,N-dimethylformamide was portionwise added sodium hydride (60% in oil, 1.18 g, 29.61 mmol) with stirring at 0°C, and iodomethane (1.85 ml, 29.61 mmol) was added thereto. The resulting solution was heated to 50°C and stirred for 3 hours, followed by stirring at 70°C for 3.5 hours. The reaction solution was neutralized with a saturated aqueous solution of ammonium chloride, and water was added thereto followed by extraction with chloroform. The resulting organic layer was washed with water and a saturated aqueous solution of sodium chloride and dried over magnesium sulfate, and the solvent was distilled off under reduced pressure to give 18.1 g of a crude product. The obtained crude product was washed with ethanol under heating to give 11.0 g (yield: 86%) of the title compound.

$^1$H-NMR($CDCl_3$) δ(ppm): 2.95 (3H, s), 6.44 (1H, s), 7.15-7.55 (17H, m), 8.09 (2H, d, J=7.6Hz), 8.22 (1H, d, J=9.2Hz).

Reference Example 3

N-Diphenylmethyl-N-isopropyl-1-benzenesulfonylindole-2-sulfonamide

To a solution of N-isopropyl-1-benzenesulfonylindole-2-sulfonamide (10.0 g, 26.42 mmol), triphenylphosphine (10.4 g, 39.63 mmol), and benzhydrol (7.30 g, 39.63 mmol) in 100 ml of tetrahydrofuran was dropwise added a solution of diethyl azodicarboxylate (6.24 ml, 39.63 mmol) in 100 ml of tetrahydrofuran with stirring, followed by stirring at room temperature for 2 hours. Water was added to the reaction mixture followed by extraction with ethyl acetate. The resulting organic layer was washed with a saturated aqueous solution of sodium chloride and dried over magnesium sulfate, and the solvent was distilled off under reduced pressure to give 34.5 g of a crude product. The obtained crude product was purified with silica gel column chromatography (ethyl acetate/hexane = 1/9) followed by crystallization from ethanol to give 11.4 g (yield: 79%) of the title compound.

$^1$H-NMR($CDCl_3$) δ(ppm): 1.27 (6H, d, J=6.6Hz), 4.6-4.75 (1H, m), 6.26 (1H, s), 6.62 (1H, s), 7.1-7.5 (16H, m), 7.93 (2H, d, J=7.3Hz), 8.22 (1H, d, J=8.6Hz).

Reference Example 4

N-Diphenylmethyl-N-[3-(2-oxopyrrolidinyl)propyl]-1-benzenesulfonylindole-2-sulfonamide

Substantially the same procedure as in Reference Example 3 was repeated using N-[3-(2-oxopyrrolidinyl)propyl]-1-benzenesulfonylindole-2-sulfonamide (10.0 g, 21.67 mmol) and benzhydrol (6.00 g, 32.50 mmol) to give 4.85 g (yield: 36%) of the title compound.

$^1$H-NMR($CDCl_3$) δ(ppm): 1.2-1.4 (2H, m), 1.8-2.0 (2H, m), 2.29 (2H, t, J=8.1Hz), 2.97 (2H, t, J=6.6Hz), 3.07 (2H, t, J=7.1Hz), 3.55-3.7 (2H, m), 6.45 (1H, s), 7.1-7.35 (7H, m), 7.35-7.6 (8H, m), 7.6-7.75 (2H, m), 8.03 (2H, d, J=7.6Hz), 8.18 (1H, d, J=8.6Hz).

Reference Example 5

N-Diphenylmethyl-N-methylindole-2-sulfonamide

To a solution of N-diphenylmethyl-N-methyl-1-benzenesulfonyl-indole-2-sulfonamide (10.70 g, 20.71 mmol)

obtained in Reference Example 2 in 100 ml of ethanol was added a 2N aqueous sodium hydroxide solution (35 ml), followed by heating under reflux for 2.5 hours. The solvent was distilled off under reduced pressure, and water and ethyl acetate were added, followed by extraction with ethyl acetate. The resulting organic layer was washed with water, a saturated aqueous solution of sodium bicarbonate, and a saturated aqueous solution of sodium chloride, and dried over magnesium sulfate, and the solvent was distilled off under reduced pressure to give 8.38 g of a crude product. The obtained crude product was washed with diethyl ether to give 7.22 g (yield: 93%) of the title compound.

$^1$H-NMR($CDCl_3$) δ(ppm): 2.73 (3H, s), 6.54 (1H, s), 6.93 (1H, d, J=2.0Hz), 7.05-7.35 (13H, m), 7.62 (1H, d, J=7.9Hz), 8.16 (1H, s).

Reference Example 6

N-Diphenylmethyl-N-isopropylindole-2-sulfonamide

Substantially the same procedure as in Reference Example 5 was repeated using N-diphenylmethyl-N-isopropyl-1-benzenesulfonylindole-2-sulfonamide (11.0 g, 20.20 mmol) obtained in Reference Example 3 to give 7.30 g (yield: 89%) of the title compound.

$^1$H-NMR($CDCl_3$) δ(ppm): 1.19 (6H, d, J=6.6Hz), 4.25-4.4 (1H, m), 5.98 (1H, s), 6.83 (1H, s), 7.0-7.2 (3H, m), 7.2-7.4 (10H, m), 7.59 (1H, d, J=7.9Hz).

Reference Example 7

N-Diphenylmethyl-N-[3-(2-oxopyrrolidinyl)propyl]indole-2-sulfonamide

Substantially the same procedure as in Reference Example 5 was repeated using N-diphenylmethyl-N-[3-(2-oxopyrrolidinyl)propyl]-1-benzenesulfonylindole-2-sulfonamide (4.80 g, 7.65 mmol) obtained in Reference Example 4 to give 2.97 g (yield: 80%) of the title compound.

$^1$H-NMR($CDCl_3$) δ(ppm): 1.35-1.55 (2H, m), 1.85-2.0 (2H, m), 2.34 (2H, t, J=7.7Hz), 2.98 (2H, t, J=6.4Hz), 3.05-3.15 (2H, m), 3.36 (2H, t, J=7.6Hz), 6.41 (1H, s), 6.96 (1H, d, J=2.0Hz), 7.0-7.1 (4H, m), 7.1-7.35 (9H, m), 7.62 (1H, d, J=8.3Hz), 8.85-9.05 (1H, m).

Reference Example 8

N-Diphenylmethyl-N-methyl-1-(2-dimethylaminoethyl)indole-2-sulfonamide

Substantially the same procedure as in Example 2 was repeated using N-diphenylmethyl-N-methylindole-2-sulfonamide (2.00 g, 5.31 mmol) obtained in Reference Example 5 and 2-dimethylaminoethylchloride hydrochloride (0.84 g, 5.84 mmol) to give 2.01 g (yield: 85%) of the title compound.

$^1$H-NMR($CDCl_3$) δ(ppm): 2.26 (6H, s), 2.55-2.65 (2H, m), 2.80 (3H, s), 4.15-4.25 (2H, m), 6.53 (1H, s), 7.00 (1H, s), 7.05-7.15 (4H, m), 7.15-7.4 (9H, m), 7.64 (1H, d, J=7.9Hz).

Reference Example 9

N-Diphenylmethyl-N-methyl-1-(2-pyrrolidinylethyl)indole-2-sulfonamide

Substantially the same procedure as in Example 2 was repeated using N-diphenylmethyl-N-methylindole-2-sulfonamide (2.00 g, 5.31 mmol) obtained in Reference Example 5 and 2-pyrrolidinylethylchloride hydrochloride (0.99 g, 5.84 mmol) to give 2.30 g (yield: 91%) of the title compound.

$^1$H-NMR($CDCl_3$) δ(ppm): 1.65-1.8 (4H, m), 2.45-2.6 (4H, m), 2.7-2.8 (2H, m), 2.80 (3H, s), 4.2-4.3 (2H, m), 6.53 (1H, s), 7.00 (1H, s), 7.0-7.15 (4H, m), 7.15-7.4 (9H, m), 7.64 (1H, d, J=7.9Hz).

Reference Example 10

N-Diphenylmethyl-N-isopropyl-1-(2-dimethylaminoethyl)indole-2-sulfonamide

Substantially the same procedure as in Example 2 was repeated using N-diphenylmethyl-N-isopropylindole-2-sulfonamide (2.0 g, 4.94 mmol) obtained in Reference Example 6 and 2-dimethylaminoethylchloride hydrochloride (0.79 g, 5.44 mmol) to give 2.05 g (yield: 87%) of the title compound.

$^1$H-NMR($CDCl_3$) δ(ppm): 1.27 (6H, d, J=6.6Hz), 2.26 (6H, s), 2.5-2.6 (2H, m), 3.9-4.05 (1H, m), 4.1-4.2 (2H, m), 6.25 (1H, s), 6.89 (1H, s), 7.1-7.2 (1H, m), 7.2-7.35 (12H, m), 7.57 (1H, d, J=8.3Hz).

Reference Example 11

N-Diphenylmethyl-N-isopropyl-1-(2-pyrrolidinylethyl)indole-2-sulfonamide

Substantially the same procedure as in Example 2 was repeated using N-diphenylmethyl-N-isopropylindole-2-sulfonamide (2.0 g, 4.94 mmol) obtained in Reference Example 6 and 2-pyrrolidinylethylchloride hydrochloride (0.93 g, 5.44 mmol) to give 2.26 g (yield: 91%) of the title compound.

$^1$H-NMR($CDCl_3$) δ(ppm): 1.27 (6H, d, J=6.9Hz), 1.65-1.8 (4H, m), 2.45-2.6 (4H, m), 2.65-2.8 (2H, m), 3.85-4.0 (1H, m), 4.15-4.25 (2H, m), 6.25 (1H, s), 6.88 (1H, s), 7.05-7.2 (1H, m), 7.2-7.35 (12H, m), 7.56 (1H, d, J=7.9Hz).

Reference Example 12

N-Diphenylmethyl-N-[3-(2-oxopyrrolidinyl)propyl]-1-(2-dimethylaminoethyl)indole-2-sulfonamide

Substantially the same procedure as in Example 2 was repeated using N-diphenylmethyl-N-[3-(2-oxopyrrolidinyl)propyl]indole-2-sulfonamide (1.40 g, 2.87 mmol) obtained in Reference Example 7 and 2-dimethylaminoethylchloride hydrochloride (0.46 g, 3.16 mmol) to give 1.23 g (yield: 77%) of the title compound.

$^1$H-NMR($CDCl_3$) δ(ppm): 1.2-1.4 (2H, m), 1.8-2.0 (2H, m), 2.2-2.35 (2H, m), 2.26 (6H, s), 2.45-2.6 (2H, m), 2.9-3.05 (4H, m), 3.2-3.35 (2H, m), 3.85-4.0 (2H, m), 6.54 (1H, s), 6.95-7.4 (14H, m), 7.64 (1H, d, J=8.3Hz).

Reference Example 13

N-Diphenylmethyl-N-[3-(2-oxopyrrolidinyl)propyl]-1-(2-pyrrolidinylethyl)indole-2-sulfonamide

Substantially the same procedure as in Example 2 was repeated using N-diphenylmethyl-N-[3-(2-oxopyrrolidinyl)propyl]indole-2-sulfonamide (1.40 g, 2.87 mmol) obtained in Reference Example 7 and 2-pyrrolidinylethylchloride hydrochloride (0.54 g, 3.16 mmol) to give 1.62 g (yield: 97%) of the title compound.

$^1$H-NMR($CDCl_3$) δ(ppm): 1.2-1.4 (2H, m), 1.8-1.95 (6H, m), 2.29 (2H, t, J=8.5Hz), 2.45-2.7 (4H, m), 2.7-2.9 (2H, m), 2.9-3.05 (4H, m), 3.2-3.35 (2H, m), 3.95-4.15 (2H, m), 6.52 (1H, s), 6.9-7.0 (4H, m), 7.09 (1H, s), 7.1-7.4 (9H, m), 7.64 (1H, d, J=7.9Hz).

Industrial Applicability

According to the present invention, there can be provided indole derivatives which are useful as therapeutic agents for osteoporosis.

## Claims

1. An indole derivative represented by formula (I):

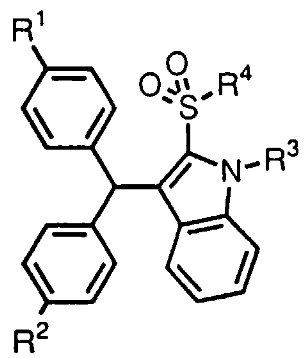

(I)

wherein $R^1$ and $R^2$ independently represent hydrogen, lower alkyl, hydroxy, lower alkoxy, halogen, $-O-(CH_2)_n-OR^5$ (wherein $R^5$ represents hydrogen or lower alkyl, and n represents an integer of 1 to 6), or

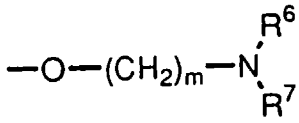

(wherein $R^6$ and $R^7$ independently represent hydrogen or lower alkyl, or $R^6$ and $R^7$ are combined together with the adjacent nitrogen atom to form a substituted or unsubstituted alicyclic heterocyclic group, and in represents an integer of 2 to 6), $R^3$ represents hydrogen, lower alkyl, or

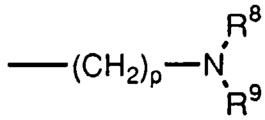

(wherein $R^8$ and $R^9$ independently represent hydrogen or lower alkyl, or $R^8$ and $R^9$ are combined together with the adjacent nitrogen atom to form a substituted or unsubstituted alicyclic heterocyclic group, and p represents an integer of 2 to 6), and $R^4$ represents hydroxy, lower alkoxy, substituted or unsubstituted aryloxy, or $-NR^{10}R^{11}$ (wherein $R^{10}$ and $R^{11}$ independently represent hydrogen, lower alkyl, alicyclic alkyl, substituted or unsubstituted aryl, a substituted or unsubstituted heterocyclic group,

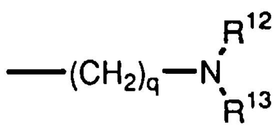

(wherein $R^{12}$ and $R^{13}$ independently represent hydrogen or lower alkyl, or $R^{12}$ and $R^{13}$ are combined together with the adjacent nitrogen atom to form a substituted or unsubstituted alicyclic heterocyclic group, and q represents an integer of 2 to 6), or $-(CH_2)_r-R^{14}$ (wherein $R^{14}$ represents substituted or unsubstituted aryl or a substituted or unsubstituted heterocyclic group, and r represents an integer of 1 to 6), or $R^{10}$ and $R^{11}$ are combined together with the adjacent nitrogen atom to form a substituted or unsubstituted alicyclic heterocyclic group), or a pharmaceutically acceptable salt thereof.

**2.** An indole derivative according to claim 1, wherein $R^1$ and $R^2$ represent hydrogen, or a pharmaceutically acceptable salt thereof.

**3.** An indole derivative according to claim 2, wherein $R^3$ represents

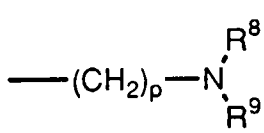

(wherein $R^8$, $R^9$, and p have the same meanings as defined above), or a pharmaceutically acceptable salt thereof.

**4.** A pharmaceutically acceptable composition comprising a pharmaceutically acceptable carrier and an effective amount of a compound according to any one of claims 1 to 3 as an active ingredient.

## INTERNATIONAL SEARCH REPORT

International application No.
PCT/JP96/01981

**A. CLASSIFICATION OF SUBJECT MATTER**

Int. Cl[6] C07D209/30, 403/06, A61K31/40, 31/445, 31/495, 31/535

According to International Patent Classification (IPC) or to both national classification and IPC

**B. FIELDS SEARCHED**

Minimum documentation searched (classification system followed by classification symbols)

Int. Cl[6] C07D209/30, 403/06, A61K31/40, 31/445, 31/495, 31/535

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| P,A | WO, 95/19343, A1 (Kyowa Hakko Co., Ltd.), July 20, 1995 (20. 07. 95), Full descriptions & AU, 9514247, A | 1 - 4 |
| A | J. Med. Chem., 17(12), (1974), p. 1298-1304, Calvert W. Whitehead et al., "Effect of lipophilic substituents on some biological properties of indoles" | 1 - 4 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| October 2, 1996 (02. 10. 96) | October 15, 1996 (15. 10. 96) |
| Name and mailing address of the ISA/ Japanese Patent Office | Authorized officer |
| Facsimile No. | Telephone No. |

Form PCT/ISA/210 (second sheet) (July 1992)